# EUROPEAN PATENT APPLICATION

(11) **EP 3 811 783 A1**
(43) Date of publication of application: **28.04.2021**
(21) Application number: 19822853.8
(22) Date of filing: 19.06.2019
(51) Int. Cl.: A23K 50/75, A23K 10/30, A23K 10/37, A61K 36/896, A61P 33/02

(54) **FEED ADDITIVE COMPOSITION COMPRISING SABADILLA SEEDS**

(30) Priority: 20.06.2018 JP 2018117000
(71) Applicant: SUMITOMO CHEMICAL COMPANY LIMITED, Chuo-ku Tokyo 104-8260 (JP)
(72) Inventor: TONOUE, Tsuyoshi, Tokyo 104-8260 (JP)
(74) Representative: Winter, Brandl - Partnerschaft mbB
(86) International application number: PCT/JP2019/024282
(87) International publication number: WO 2019/244929

(57) **Abstract**

Improved and safer products for preventing or alleviating coccidiosis, especially those that do not trigger the development of resistance are provided. A feed additive composition for preventing poultry coccidiosis comprising sabadilla seed, defatted sabadilla extract and/or extraction residue of sabadilla seed, a feed comprising said feed additive composition, and a method for preventing poultry coccidiosis using sabadilla seed, defatted sabadilla extract and/or extraction residue of sabadilla seed.

## Description

### TECHNICAL FIELD

The present invention relates to a feed additive for poultry, more particularly, to an additive for preventing poultry coccidiosis by addition to a feed. More particularly, the present invention relates to a feed additive composition for preventing poultry coccidiosis comprising sabadilla seed, defatted sabadilla extract and/or extraction residue of sabadilla seed, a feed comprising said feed additive composition, and a method for preventing poultry coccidiosis using sabadilla seed, defatted sabadilla extract and/or extraction residue of sabadilla seed.

### BACKGROUND ART

One of the most important diseases in poultry is coccidiosis because it is spread over all continents and causes heavy economic losses to the poultry industry.

Coccidiosis causes severe pathology in the intestines and ceca of poultry. It affects mainly the upper, middle, and lower small intestine, the rectum, the colon, and the ceca. Coccidiosis is caused by infections with protozoans of the genus *Eimeria* (called coccidia). The common problem causing species in poultry (chickens, turkeys, or the like), which are *Eimeria acervulina, E. brunett, E. maxima, E. mivati, E. necatrix* and E. *tenella* in chickens, which are *E. adenoeides, E. dispersa, E. gallopavonis* and *E. meleagridis* in turkeys, which are *E. aythyae,* and *E. burcephalae* in ducks. The habitats of the *Eimeria* in avian, depends on the species of *Eimeria* and the poultry species. The coccidian can inhabit the upper, middle, and lower small intestine, the rectum, the colon, the kidneys, and the ceca.

A coccidia proliferates in the mucosa of the gastrointestinal tract and excretes an infectious form covered with a hard shell called "oocyst" in the feces. The coccidia spreads to another animal by oral infection with this excreted oocyst.

For the symptoms of coccidiosis, though they may vary depending on the kind of infected coccidia, hemorrhagic lesion is formed in ceca and small intestine and symptoms such as diarrhea and bloody stool are manifested. Severe symptoms may be lethal or, even if they are not lethal, growth is greatly inhibited to adversely affect subsequent productivity. Coccidiosis is, therefore, a disease of great economic importance and extensive work has been done to find new and improved methods for controlling and treating coccidial infections in poultry.

In the prevention or treatment of coccidiosis, an antibiotic (a polyether antibiotic such as salinomycin, or the like), a chemotherapeutic agent composed of a synthetic antimicrobial agent (a sulfa drug, or the like) and a biological drug such as a vaccine have been mainly used. However, such an antibiotic or a chemotherapeutic agent has problems such as the occurrence of side effects and a decline in the effect due to acquisition of resistance to the agent, and a vaccine is used only for the prevention and cannot be used for the treatment. Moreover, in the case where humans take the meat, milk, or the like of the animal to which such an agent has been administered, there is a problem of migration in the human body of the agent remaining in the animal body, and a strict limitation on the using amount and administration period thereof has been required.

Furthermore, there are health and safety concerns with some of these coccidiostats because of overuse of antibiotics and cross-resistance. Therefore, not all of them will be available in the future, world-wide. Currently, several coccidiostats are already banned in the European Union, including amprolium and meticlorpindol.

The development of resistance against chemicals is a severe problem in monocellular eukaryotic organisms. The major reason for this is because such organisms replicate very fast and can rapidly adapt their metabolism to environmental changes. This is especially true for the protozoans that cause coccidiosis in poultry, where the chemicals are administered prophylactically all over the year, and the pathogen is permanently confronted with the chemical. In such an environment resistance can develop very fast.

This is why there is a strong need for improved and safer naturally occurring controlling agents for coccidiosis that do not trigger the development of resistance.

A series of coccidiosis preventing or alleviating agents each containing, as an active ingredient, cashew nut shell liquid and/or anacardic acids, which are main components of the cashew nut shell liquid, have been proposed (Patent Documents 1 to 5).

As a feed additive for preventing or alleviating coccidiosis, an extract of *Sanguinaria canadensis* (poppy plant) and alkaloid (Patent Document 6), gluconic acids (Patent Document 7), trypsin inhibitor (Patent Document 8), microorganisms (Patent Document 9), *Simarouba amara* dried bark or an extract thereof and/or *Momordica charantia* fruit extracts (Patent Document 10), fermented wheat germ extract (Patent Document 11), a protease and an inner salt of a quaternary amine carboxylic acid (Patent Document 12), a carbanilide compound (Patent Document 13), a fermentation product of a lactic acid bacterium and a fermentation product of a propionic acid bacterium (Patent Document 14), and deoxynarasin antibiotic complex (Patent Document 15) are known.

Plant of the genus *Schoenocaulon* is generally called sabadilla. From many tissues of sabadilla, effective naturally occurring pesticides have been obtained. Among these, the species of the longest history are *Schoenocaulon officinale.*

Alkaloids contained in sabadilla seeds are known (Patent Document 16, 17, 18, 19, and 20, and non-patent reference 1) to be effective as a pesticide and are commercially available as VeratranD™.

Non-patent reference 2 discloses that, to reduce the incidence frequency of coccidia oocysts in the intestinal tract, three different additives, i.e. homeopatics (comprising as a part sabadilla), probiotics and prebiotics, were mixed in 20 ml of water for oral administration for four weeks and as a result homeopatics reduced significantly the incidence frequency of coccidia oocysts in the intestinal tract as compared to the control and discloses the positive effect of homeopathic remedies on health status and reduction of pathogens incidence.

### PRIOR ART

### [Patent reference]

Patent reference 1: JP 2003-238400
Patent reference 2: JP 2001-151675
Patent reference 3: JP 8-231410
Patent reference 4: JP 2009-139468
Patent reference 5: JP 2010-143627
Patent reference 6: DE 4303099
Patent reference 7: JP 2008-283893
Patent reference 8: JP 08-040935
Patent reference 9: JP 2015-530876
Patent reference 10: JP 2007-520515
Patent reference 11: JP 2005-535325
Patent reference 12: JP 2003-516133
Patent reference 13: JP 09-510457
Patent reference 14: JP 2010-024413
Patent reference 15: US 4,174,404
Patent reference 16: WO 2017070437
Patent reference 17: WO 2017070440
Patent reference 18: WO 2017070444
Patent reference 19: WO 2017070447
Patent reference 20: WO 2017070451

### [Non-patent reference]

Non-patent reference 1: Journal of Economic Entomology, Vol.90, no.2, p.326-332
Non-patent reference 2: Acta fytotechn zootechn, 19, 2016(2): 51-53

### DISCLOSURE OF THE INVENTION

### (Technical Problem to be Solved by the Invention)

As mentioned above, there is a need for improved and safer products for preventing or alleviating coccidiosis, especially those that do not trigger the development of resistance.

### (Means for Solving the Problems)

Surprisingly, the present inventor has found that sabadilla seed, defatted sabadilla extract and/or extraction residue of sabadilla seed act as coccidiostats and can be effectively used for prevention of coccidiosis.

Thus, the present invention provides a feed additive composition for preventing poultry coccidiosis comprising sabadilla seed, defatted sabadilla extract and/or extraction residue of sabadilla seed, a feed comprising said feed additive composition, and a method for preventing poultry coccidiosis using sabadilla seed, defatted sabadilla extract and/or extraction residue of sabadilla seed.

Accordingly, the present invention includes the following.
[1] A feed additive composition for preventing poultry coccidiosis comprising sabadilla seed, defatted sabadilla extract and/or extraction residue of sabadilla seed.
[2] The feed additive composition according to [1] wherein the composition further comprises herbs.
[3] A feed comprising the feed additive composition of [1] or [2].
[4] The feed according to [3] wherein sabadilla seed, defatted sabadilla extract and/or extraction residue of sabadilla seed are present in the feed at a concentration of 0.125% or less, 0.045% or less, or 1.25% or less by weight, respectively.
[5] The feed according to [3] or [4] wherein herbs are present in the feed at a concentration of 0.05% to 5% by weight.
[6] The feed according to any of [3], [4] or [5] wherein the feed further comprises at least one selected from the group consisting of corn, milo, bran, rice bran, soybean meal, corn meal, rice flour and soy flour.
[7] A method for preventing coccidiosis which comprises feeding poultry the feed additive composition for preventing poultry coccidiosis of [1] or [2], or the feed of any of [3], [4], [5] or [6].
[8] A method for breeding poultry which comprises feeding poultry the feed of any of [3], [4], [5] or [6].
[9] Use of sabadilla seed, defatted sabadilla extract and/or extraction residue of sabadilla seed for prevention of coccidiosis.

### EFFECTS OF THE INVENTION

The present invention allows for safe, effective and easy prevention of coccidiosis in poultry by using sabadilla seed, defatted sabadilla extract and/or extraction residue of sabadilla seed.

### BEST MODE FOR CARRYING OUT THE INVENTION

The present inventor has found unexpectedly that coccidiosis could be prevented by mixing sabadilla seed, defatted sabadilla extract and/or extraction residue of sabadilla seed with feed of poultry.

Sabadilla seed, defatted sabadilla extract and/or extraction residue of sabadilla seed can be obtained from any species belonging to *Schoenocaulon,* which includes the following species: *Schoenocaulon calcicola, Schoenocaulon caricifolium, Schoenocaulon comatum, Schoenocaulon conzattii, Schoenocaulon dubium* (alternatively *Schoenocaulon gracile*), *Schoenocaulon framei, Schoenocaulon ghiesbreghtii* (alternatively *Schoenocaulon drummondii), Schoenocaulon yucatanense, Schoenocaulon ignigenum, Schoenocaulon intermedium, Schoenocaulon jaliscense, Schoenocaulon macrocarpum* (alternatively *Schoenocaulon lauricola*), *Schoenocaulon madidorum, Schoenocaulon megarrhizum, Schoenocaulon mortonii, Schoenocaulon oaxacense, Schoenocaulon obtusum, Schoenocaulon officinale, Schoenocaulon pellucidum, Schoenocaulon plumosum, Schoenocaulon pringlei, Schoenocaulon rzedowskii, Schoenocaulon tenorioi, Schoenocaulon tenue, Schoenocaulon tenuifolium, Schoenocaulon texanum* and *Schoenocaulon tigrense.*

Defatted sabadilla extract can be extracted from sabadilla seed by the extraction procedures described in WO2017/070437. Namely, after sabadilla seed is pulverized, seed/plant parts solvent is added to produce an oil solution and a residue. Further, an extract solvent is added to a crude oil obtained by distillation of methanol from the oil solution to produce a crude solution, which is then decanted to give an extract which is defatted sabadilla extract. Extraction of crude sabadilla oil from sabadilla seed produces a residue of sabadilla seed. The residue is referred to as extraction residue of sabadilla seed.

An alternative method for extraction is described in WO2017/070437 and comprises milling sabadilla seeds or other plant parts, washing the milled sabadilla seeds or other plant parts with at least one seed or plant part solvent selected from the group consisting of methanol, ethanol, glycol ether, ethyl lactate, propanol, butyl lactate, gamma-butyrolactone, and 1-butanol to produce a sabadilla oil solution, removing the sabadilla oil and a residue from the washed milled seeds, removing the seed and plant part solvent from the oil solution to produce a crude sabadilla oil, washing the crude sabadilla oil with at least one extract solvent selected from the group consisting of C5 to C14 alkanes, chlorinated methane, chlorinated ethane, benzene, and benzene derivatives under agitation to produce a crude sabadilla solution, removing the sabadilla solution and defatted sabadilla extract from the crude solution, and removing the extract solvent from the solution to produce sabadilla oil.

Defatted sabadilla extract can be obtained from any part of *Schoenocaulon officinale,* preferably from seeds.

As used herein, "extract solvent" refers to C5 to C14 alkanes, chlorinated methane, chlorinated ethane, benzene, and/or benzene derivatives. As used herein, "benzene derivatives" refers to a chemical compound derived from benzene wherein one or more hydrogen atoms are replaced with another functional group. Examples of benzene derivatives include phenol, toluene, and aniline. In a preferred embodiment, the extract solvent is hexane.

As used herein, the "seed or plant part solvent" refers to methanol, ethanol, glycol ether, ethyl lactate, propanol, butyl lactate, gamma-butyrolactone, and/or 1-butanol. In a preferred embodiment, the seed or plant part solvent is selected from the group consisting of methanol, ethanol, glycol ether, ethyl lactate, and propanol. In a more preferred embodiment, the seed or plant part solvent is methanol.

Methanol can be used at temperatures from about 0° to about 60°C. Methanol at lower temperatures requires additional extraction time and temperatures above about 55° to about 60°C result in methanol loss and boiling. The optimal temperature for methanol extraction is from about 50° to about 55°C.

As used herein, "sabadilla oil solution" refers to a solution containing at least one "seed or plant part solvent" selected from the group consisting of methanol, ethanol, glycol ether, ethyl lactate, propanol, butyl lactate, gamma-butyrolactone, and 1-butanol and the parts of the seeds which dissolve in seed or plant part solvent, such as the resins, alkaloids and oil. This solvent extraction separates the resins, alkaloids and oils from the cellulose, hemicellulose, lignin and pectin of the seeds.

As used herein, "crude sabadilla extract" refers to what is left after the seeds have been washed with at least one solvent selected from the group consisting of methanol, ethanol, glycol ether, ethyl lactate, propanol, butyl lactate, gamma-butyrolactone, and 1-butanol (seed or plant part extraction) and the seed or plant part solvent has been removed from oil solution. The lipid profile of sabadilla oil is generally about half triglycerides, followed by diglycerides, plant waxes and monoglycerides. Free fatty acids and sterols are normally also present as minor constituents. The overall fatty acid profile normally contains fatty acids from C-12 to C-24, but is usually dominated by C-18, with the monounsaturated oleic acid (C-18: 1) normally the major proportion the C-18 acids.

As used herein, "crude sabadilla solution" refers to a solution containing at least one extract solvent selected from the group consisting of C5 to C14 alkanes, chlorinated methane, chlorinated ethane, benzene, and benzene derivatives and the oils from the sabadilla seeds which dissolve in the extract solvent(s). This solvent extraction separates the oils from the rest of the defatted extract or parts of the seeds.

As used herein, the expression "poultry" is used for all sorts of breeds of domesticated birds independent of their age, comprising chickens, quails, ducks, geese, pigeons, turkeys, and ostriches. The expression "poultry" is preferably used for chickens, ducks, geese, and turkeys, and most preferably for chickens. Chickens include broilers and layers.

For the feed additive composition for preventing poultry coccidiosis of the present invention, sabadilla seed, defatted sabadilla extract and/or extraction residue of sabadilla seed may be used as they stand. Alternatively, sabadilla seed and defatted sabadilla extract may be added with excipients such as a solid carrier and liquid carrier to prepare formulations of tablets, powders, granules, capsules, water soluble powders, liquids, wettable powders, or suspensions. Solid carrier excipients include, for instance, lactose, sucrose, glucose, cornstarch, gelatin, casein, starch, acacia, cellulose derivative, alginic acid, and the like. Liquid carrier excipients include, for instance, water, glycerin, vegetable oil, fatty acid, fatty acid ester, sorbitol, and the like.

The feed additive composition for preventing poultry coccidiosis of the present invention may further comprises proteins, vitamins (vitamin A, vitamin B1, vitamin B2, vitamin B6, vitamin 12, vitamin D, vitamin E, calcium pantothenate, nicotinic acid, folic acid, etc.) and trace inorganic salts (magnesium sulfate, iron sulfate, copper sulfate, zinc sulfate, potassium iodide, cobalt sulfate, etc.), and the like. The feed additive composition for preventing poultry coccidiosis of the present invention may also be supplemented with additives such as antioxidants, flavoring agents, and dyes.

The feed additive composition for preventing poultry coccidiosis of the present invention may be used as it stands or may be added to feed for poultry. The feed additive composition for preventing poultry coccidiosis of the present invention may be mixed with, or used together with, additives such as antioxidants, flavoring agents, and dyes.

The way of administration of the feed additive composition for preventing poultry coccidiosis of the present invention is not particularly limited but it is easy and preferable to mix the composition with feed for poultry and to apply the mixture to poultry. A dose is an amount effective for prevention of coccidiosis in poultry such that, when the other conditions are unchanged, a preventing effect for coccidiosis is promoted by administration of the feed additive composition for preventing poultry coccidiosis of the present invention in comparison with no administration thereof. Specifically, sabadilla seed thereof is added at a concentration of 0.005% to 0.125%, preferably 0.01% to 0.1%, more preferably 0.03% to 0.07% by weight, defatted sabadilla extract thereof is added at a concentration of 0.0018% to 0.045%, preferably 0.0036% to 0.036%, more preferably 0.0011% to 0.0252% by weight, extraction residue of sabadilla seed is added at a concentration of 0.05% to 1.25%, preferably 0.1% to 1.0%, more preferably 0.3% to 0.7% by weight. Preferably, sabadilla seed, defatted sabadilla extract and/or extraction residue of sabadilla seed is added at a concentration of 0.125% or less, 0.045% or less, or 1.25% or less by weight in feed, respectively.

Feed for poultry to be mixed with the feed additive composition for preventing poultry coccidiosis of the present invention is not particularly limited and may be the ones that are commonly used. Specifically, the feed additives composition is added to at a concentration of 0.005% to 1.0%, preferably 0.05% to 0.5%, more preferably 0.1% to 0.3% by weight in the feed. An example includes a feed prepared by suitably mixing corn, rice, wheat, milo, soybean meal, rapeseed meal, bran, defatted rice bran, fish meal, skimmed milk powder, dried whey, alfalfa meal, Hokuyo meal, soybean oil, powdered refined tallow, wheat flour, rapeseed oil, meat bone meal (feather meal), animal fats and oils, calcium phosphate, corn gluten meal, corn distiller's grain solubles, molasses, calcium carbonate, tricalcium phosphate, sodium chloride, choline chloride, vitamins (vitamin A, vitamin B1, vitamin B2, vitamin B6, vitamin 12, vitamin D, vitamin E, calcium pantothenate, nicotinic acid, folic acid, etc.), amino acids (lysine, methionine, threonine, etc.), trace inorganic salts (magnesium sulfate, iron sulfate, copper sulfate, zinc sulfate, potassium iodide, cobalt sulfate, etc.), organic acids (formic acid, propionic acid, etc.), enzymes (phytase, etc.), probiotics, and the like. The feed additive composition of the present invention and the feed of the present invention preferably comprises herbs and/or at least one selected from the group consisting of corn, milo, bran, rice bran, soybean meal, corn meal, rice flour and soy flour.

For the herbs, *Acorus calamus, Allium sativum, Anethum graveolens, Artemisia absinthium, Carum carvi, Cinnamomum, Coriandrum sativum, Cuminum cyminum, Curcuma longa, Cymbopogon citratus, Cynara scolymus, Echinacea, Elettaria cardamomum, Foeniculum vulgare, Ginkgo biloba, Glycyrrhiza glabra, Hypericum perforatum, Laurus nobilis, Levisticum officinale, Melaleuca alternifolia, Melissa officinalis, Mentha spicata, Mentha x piperita, Myristica fragrans, Ocimum basilicum, Origanum majorana, Origanum vulgare, Panax ginseng, Petroselinum sativum, Pimenta dioica, Pimpinella anisum, Piper nigrum, Pogostemon cablin, Rosmarinus officinalis, Salvia officinalis, Stevia rebaudiana, Syzygium aromaticum, Tanacetum vulgare, Taraxacum officinale, Thymus, Trachyspermum ammi, Zingiber officinale,* and the like may be used each alone or in admixture thereof or in combination thereof. Preferable herbs include *Acorus calamus, Allium sativum, Artemisia absinthium, Cinnamomum, Echinacea, Origanum vulgare, Syzygium aromaticum* and *Thymus.* These herbs may be used each alone or in admixture thereof or in combination thereof. Allium sativum is the most preferable.

The herbs may be used in the form of powder or may be extracted and used in the form of an essential oil and the like.

A content of the herbs in the feed of the present invention is not particularly restricted but may generally be in the range of 0.05% to 5% by weight, preferably 0.1% to 3% by weight, more preferably 0.25% to 1% by weight.

In case that a feed contains sabadilla seed and herbs, a ratio of the weight of herbs to the weight of sabadilla seed is usually 10 or more and 50 or less, preferably 20 or more and 40 or less, more preferably 25 or more and 35 or less.

In case that a feed contains defatted sabadilla extract and herbs, a ratio of the weight of herbs to the weight of defatted sabadilla extract is usually 28 or more and 139 or less, preferably 56 or more and 111 or less, more preferably 69 or more and 97 or less.

In case that a feed contains extraction residue of sabadilla seed and herbs, a ratio of the weight of herbs to the weight of extraction residue of sabadilla seed is usually 1 or more and 5 or less, preferably 2 or more and 4 or less, more preferably 2.5 or more and 3.5 or less.

Administration time of the feed additive composition for preventing poultry coccidiosis or the feed comprising the composition of the present invention is all breeding stage, preferably young chicken stage (0 to 5 weeks old after hatching). More preferably, it is administered to poultry continually for 0 to 21 days after hatching.

The present invention is explained in more detail with the following examples but is not limited thereto.

### [Example 1]

Sabadilla seeds were flake milled according to the manufacturer's instructions. Two hundred grams of milled seed were added to a three liter flask with methanol at 40°C to 45°C and stirred with a three blade stirrer controlled by an overhead motor. A stirring speed was maintained which prevented any seed fragments from settling in the flask. The system was sealed to limit evaporation loss.

The sabadilla oil solution was decanted off and additional methanol was added to the flask. This step was repeated three additional times. The washed milled seeds (extraction residue of sabadilla seed) remained in the bottom of the flask when the sabadilla oil solution was decanted.

The decanted sabadilla oil solution was placed in a new flask. Distillation was then used to remove the methanol from the sabadilla oil solution. Standard IKA rotary evaporators were used for the distillation. The flask containing the sabadilla oil solution was loaded into the evaporator and into a heated water bath. The flask was heated to between 50°C to 55°C in order to maximize efficient removal of the methanol without allowing it to boil over into the condenser. The evaporated methanol was thoroughly condensed in an adjoining flask leaving the sabadilla crude oil. Thus, the sabadilla crude oil with high purity was prepared.

Accordingly, a sabadilla crude oil was prepared which has had the seed components, such as cellulose, removed from the milled seed. The sabadilla crude oil was then added to a three liter flask with hexane at 40°C to 45°C and stirred with a three blade stirrer controlled by an overhead motor. A stirring speed was maintained which prevented any sediment from settling in the flask. The system was sealed to limit evaporation loss.

The sabadilla solution was decanted off and additional hexane was added to the flask. This step was repeated three additional times for all traces of the solution from the milled seed. A total of two liters of hexane was used. Sediment (defatted sabadilla extract) remained in the flask when the sabadilla oil solution was decanted off.

### [Example 2]

### Test for estimating anti-coccidial effect of sabadilla

As feed material, a feed supplemented with sabadilla seed as well as extraction residue of sabadilla seed obtained in Example 1 was given to broiler chickens of 8 days old. At 10 days old, the broiler chickens were forced to receive a single dose of fresh sporulated oocyst of chicken coccidia (*E. tenella*). Anti-coccidial effect of said feed material was estimated from nature of feces, OPG (oocysts per gram of feces) and intestinal lesion of broiler chickens up till 18 days old.

### Material and method

### 1) Animals and substance tested

Animals tested: Fifty ROSS broiler chickens proved to be normal during 8-day habituation period starting from 0 days old were used for the test. The animals were assigned to each group when they were 8 days old (5 animals/test area; 10 test areas in total).

### Substance tested: Sabadilla seed and its exraction residue (Schoenocaulon officinale)

### 2) Test period

### Habituation period: 0 to 7 days old

### Period for provision of feed material: 8 to 18 days old

### 3) Test area

Ten test areas were provided as follows:

**[Table 1]**

| Test area | Substance added | Concentration | Coccidial infection | Number of chickens |
|---|---|---|---|---|
| 1 | Basal feed alone | - | - | 5 |
| 2 | Basal feed alone | - | + | 5 |
| 3 | Sabadilla seed | 0.050% | + | 5 |
| 4 | | 0.025% | + | 5 |
| 5 | | 0.010% | + | 5 |
| 6 | | 0.005% | + | 5 |
| 7 | Extraction residue of sabadilla seed | 0.50% | + | 5 |
| 8 | | 0.25% | + | 5 |
| 9 | | 0.10% | + | 5 |
| 10 | | 0.05% | + | 5 |

### 4) Breeding management

Each breeding chamber was temperature-controlled with an air conditioner. Temperature of the breeding chamber was measured and recorded with a thermo-hygrometer installed in the center of the breeding chamber. A compound feed for SD broiler fattening early stage manufactured by FEED ONE CO., LTD. was used as a basal feed. The broiler chickens were restrictedly given the feed. Basal feed components were as follows:

**[Table 2]**

| Component | |
|---|---|
| Crude protein | 23.0% or more |
| Crude fat | 5.0% or more |
| Crude fiber | 4.0% or more |
| Crude ash | 6.5% or more |
| Calcium | 0.70% or more |
| Phosphate | 0.55% or more |
| Metabolic energy | 3,000kcal/kg or more |

Feed additives contained in the basal feed were as follows: corn, dextrin, soy bean meal, white fish meal, animal fats and oils, alfalfa meal, calcium phosphate, calcium carbonate, salt, lactose, vitamin A, vitamin D3, vitamin E, vitamin K, vitamin B1, vitamin B2, vitamin B6, pantothenic acid, biotin, nicotinic acid, vitamin B12, folic acid, choline, methionine, iron sulfate, copper sulfate, zinc sulfate, cobalt sulfate, zinc carbonate, manganese sulfate, calcium iodide, casein sodium, and an emulsifying agent.

During preliminary breeding period (0 to 7 days old), the broiler chickens were fed with basal feed. During 11 days (8 to 18 days old), the broiler chickens were fed with feed with addition of the respective test material at a specific concentration. The broiler chickens received drinking water freely by a waterer with a picker.

### 5) Observation and items to be measured

(i) Nature of feces: From the previous day (9 days old) of forced oral administration of oocyst until the end of the test (18 days old), nature (normal, loose feces, watery feces, blood feces) after mixture was observed and recorded every day.
(ii) Discharged amount of oocyst: Measurement was made every day (12 to 18 days old) two days after forced oral administration of oocyst. All the feces of the respective test areas were mixed. Two grams of the mixture were taken, diluted 20-fold with 0.5% Tween 20 and stirred with a stirrer for about 30 minutes. The feces solution obtained immediately after stirring was taken to a plankton counter to count oocyst. In case that counting is difficult due to exceeding number of oocysts, the feces solution was further diluted 10-fold for counting of oocysts. Using the counted number of oocysts, the number of oocysts per 1 g of feces was measured. The number of oocysts was measured three times for each of the test areas.
(iii) Intestinal lesion score: At the end of the test, all the animals were anatomized to observe the cecum. The intestinal lesion was scored and recorded in accordance with following criteria of scoring.
   0: The cecum is utterly normal.
   1: The shape of the cecum is normal. Its content is slightly fluid and has a yellowish color. The membrane of the cecum is partially of light swelling and looks whitish.
   2: The shape of the cecum is almost normal. Swelling of allover the mucosal membrane is seen. Its content has no bleeding and a mucous solution is yellowish and faded. Within the mucous membrane, a small number of white petechial necrotic focus and hemorrhagic lesion are seen.
   3: Atrophy and deformation of the cecum is apparent. No normal content is present. The content is likely to be filled with clotting blood or grey-white cheese-like denatured products. The cecum wall is extremely thickened and fragile and petechial hemorrhagic lesion sometimes remains. The lesion reaches the cecum base but not the rectum.
   4: Atrophy and deformation of the cecum is prominent. The cecum looks like sausage and is slightly longer than or smaller than the rectum. The lesion sometimes reaches 1/3-1/4 of the rectum. Even if the lesion does not reach the rectum, the degree of 4 will apply when atrophy is extreme. The others are the same as those of 3.

### Statistical analysis

For the intestinal lesion score, mean values and standard deviation (SD) of the respective test areas were calculated. Statistical analysis was conducted for the respective additives with a test area of coccidial infection and no addition (test area 2) being control group. For the intestinal lesion score, Kruskal-Wallis test was conducted. As a result, in case of P<0.05, significant difference between the test areas was studies using Steel multiple comparison test.

### Results

The intestinal lesion score and OPG on the day when the test was ended are shown in Tables 3 and 4, respectively. In cased of a test area of no coccidial infection and no addition, nature of feces was normal and no discharge of oocysts was seen throughout the test period. Also, autopsy at the end of the test showed that the cecum was normal (lesion score: 0) for all the animals. In case of a control test area of coccidial infection and no addition, a very little amount of mucous and bloody feces was observed in normal feces on Day 3 after the forced oral administration (infection) of oocysts (mucous and bloody/normal), normal feces was observed on Day 4, blood feces was observed on Days 5 to 7, and a very little amount of mucous and bloody feces was observed in loose feces on Day 8. Oocyst discharge was observed on Days 7 and 8 after infection with OPG being 163 and 164 × 10⁴/g, respectively. Also, autopsy at the end of the test showed that the lesion score of the cecum was 4 for all the animals.

### 1) Addition of sabadilla seed

The intestinal lesion score and OPG for a control test area of coccidial infection and no addition and four test areas of addition of sabadilla seed are summarized in the following table (mean value ± SD).

**[Table 3]**

| Concentration (%) | Intestinal lesion score (0 to 4) | OPG (×10⁴/g) ^{a)} | |
|---|---|---|---|
| | | Day 7 after infection | Day 8 after infection |
| 0 | 4.0±0.0 | 163 | 164 |
| 0.050 | 2.2±0.4* | 149 | 103 |
| 0.005 | 2.8±0.4* | 72 | 81 |

| | | | |
|---|---|---|---|
| a) Mean values of three tests are shown. *: Significantly different from the group of coccidial infection and no addition (P<0.05) | | | |

A very little amount of mucous and bloody feces was observed in normal feces in a portion of the concentration areas on Day 2 after infection, blood feces were observed in almost all of the concentration areas on Days 5 to 7, and a very little amount of blood feces was observed in loose feces on Day 8.

### 2) Addition of extraction residue of sabadilla seed

The intestinal lesion score and OPG for a control test area of coccidial infection and no addition and four test areas of addition of extraction residue of sabadilla seed are summarized in the following table (mean value ± SD).

**[Table 4]**

| Concentration (%) | Intestinal lesion score (0 to 4) | OPG(×10⁴/g)^{a)} | |
|---|---|---|---|
| | | Day 7 after infection | Day 8 after infection |
| 0 | 4.0±0.0 | 163 | 164 |
| 0.50 | 2.6±0.5* | 82 | 133 |
| 0.05 | 2.8±0.4* | 159 | 81 |

| | | | |
|---|---|---|---|
| a) Mean values of three tests are shown. *: Significantly different from the group of coccidial infection and no addition (P<0.05) | | | |

A very little amount of mucous and bloody feces was observed in normal feces in almost all the concentration areas on Day 3 after infection, blood feces were observed in almost all of the concentration areas on Days 5 to 7, and almost no blood feces was observed on Day 8.

As mentioned above, alleviation of the intestinal lesion by coccidial infection and reduction of OPG were observed in broiler chickens fed with a feed supplemented with sabadilla seed, defatted sabadilla extract and/or extraction residue of sabadilla seed to prove an anti-coccidial effect.

### [Example 3]

### Test for estimating anti-coccidial effect of mixture of sabadilla and herb

A feed supplemented with sabadilla seed and powdered *Allium sativum* was given to broiler chickens of 0 day old. At 21 days old, the broiler chickens were forced to receive a single dose of fresh sporulated oocyst of chicken coccidia (*E. tenella*). OPG (oocysts per gram of the content of the cecum) in the content of the cecum was measured at 28 days old to estimate anti-coccidial effect of the mixture of sabadilla seed and herb.

### Material and method

### 1) Animals and substance tested

Animals tested: 126 ROSS broiler chickens proved to be normal during 21-day habituation period starting from 0 days old were used for the test. The animals were assigned to each group when they were 0 days old (14 animals/repeat; 3 repeats/test area; 3 test areas in total).

### Substance tested: Sabadilla seed (Schoenocaulon officinale) and powdered Allium sativum

### 2) Test period

### Habituation period: 0 to 21 days old

### Period for provision of feed material: 0 to 28 days old

### 3) Test area

Three test areas were provided as follows:

**[Table 5]**

| Test area | Substance added | Concentration | Coccidial infection | Number of chickens |
|---|---|---|---|---|
| 1 | Basal feed alone | - | + | 42 |
| 2 | Sabadilla seed | 0.0167% | + | 42 |
| 3 | Sabadilla seed + powdered *Allium sativum* | Sabadilla seed: 0.0167% powdered *Allium sativum*: 0.5% | + | 42 |

### 4) Breeding management

Each breeding chamber was temperature-controlled with a gas brooder and a warmer. Temperature of the breeding chamber was measured with a thermo-hygrometer and recorded. A compound feed for SD broiler fattening early stage manufactured by FEED ONE CO., LTD. was used as a basal feed as used in Example 2. The broiler chickens received the feed freely.

### 5) Measurement

OPG in the content of cecum: On Day 28 after the forced oral administration of oocyst, each two chickens from each repeat of the respective test area (six chickens in total) were subject to autopsy. The content of the cecum (2 g) was taken from each chicken, diluted 20-fold with 0.5% Tween20 and stirred with a stirrer for about 30 minutes. The solution of the cecum content obtained immediately after stirring was taken to a McMaster counter to count oocyst. Using the counted number of oocysts, the number of oocysts per 1 g of the cecum content was calculated.

### Results

OPG on Day 28 is shown in Table 6. OPG in the cecum content of a control test area of coccidial infection with no addition was 6.84 × 10⁴/g. On the other hand, OPG of test areas of coccidial infection with addition of 0.0167% sabadilla seed was 6.71 × 10⁴/g, and OPG of test areas of coccidial infection with addition of a mixture of 0.0167% sabadilla seed and 0.5% powdered *Allium sativum* was 5.89 × 10⁴/g.

**[Table 6]**

| Test area | Substance added | Concentration | OPG^{a)} in the cecum content (×10⁴)/g |
|---|---|---|---|
| 1 | Basal feed alone | - | 6.84 |
| 2 | Sabadilla seed | 0.0167% | 6.71 |
| 3 | Sabadilla seed + powdered *Allium sativum* | Sabadilla seed: 0.0167% powdered *Allium sativum*: 0.5% | 5.89 |

| | | | |
|---|---|---|---|
| a): Mean of cecum content of 6 chickens | | | |

As mentioned above, reduction of OPG by coccidial infection was observed in broiler chickens fed with a feed supplemented with a mixture of sabadilla seed and powdered *Allium sativum* to prove an anti-coccidial effect.

### INDUSTRIAL APPLICABILITY

A feed additive composition for preventing poultry coccidiosis comprising sabadilla seed, defatted sabadilla extract and/or extraction residue of sabadilla seed in accordance with the present invention can effectively be used for prevention of coccidiosis in poultry.

## Claims

1. A feed additive composition for preventing poultry coccidiosis comprising sabadilla seed, defatted sabadilla extract and/or extraction residue of sabadilla seed.

2. The feed additive composition according to claim 1 wherein the composition further comprises herbs.

3. A feed comprising the feed additive composition of claim 1 or claim 2.

4. The feed according to claim 3 wherein sabadilla seed, defatted sabadilla extract and/or extraction residue of sabadilla seed are present in the feed at a concentration of 0.125% or less, 0.045% or less, or 1.25% or less, respectively.

5. The feed according to claim 3 or 4 wherein herbs are present in the feed at a concentration of 0.05% to 5% by weight.

6. The feed according to any of claim 3, 4 or 5 wherein the feed further comprises at least one selected from the group consisting of corn, milo, bran, rice bran, soybean meal, corn meal, rice flour and soy flour.

7. A method for preventing coccidiosis which comprises feeding poultry the feed additive composition for preventing poultry coccidiosis of claim 1 or 2, or the feed of any of claim 3, 4, 5 or 6.

8. A method for breeding poultry which comprises feeding poultry the feed of any of claim 3, 4, 5 or 6.

9. Use of sabadilla seed, defatted sabadilla extract and/or extraction residue of sabadilla seed for prevention of coccidiosis.
